# EUROPÄISCHE PATENTSCHRIFT

(11) **EP 2 355 815 B1**
(45) Veröffentlichungstag und Bekanntmachung des Hinweises auf die Patenterteilung: **06.09.2017**
(21) Anmeldenummer: 09760506.7
(22) Anmeldetag: 12.11.2009
(51) Int. Cl.: A61K 31/381, A61K 31/404, A61K 31/41, A61K 31/47, A61K 45/06, A61P 11/00

(54) **VERWENDUNG VON LEUKOTRIEN-INHIBITOREN ZUR BEHANDLUNG VON LUNGENERKRANKUNGEN BEI FRÜHGEBORENEN KINDERN**
USE OF LEUKOTRIENE INHIBITORS FOR TREATING LUNG DISEASES IN PREMATURELY BORN INFANTS
UTILISATION D'INHIBITEURS DES LEUCOTRIÈNES POUR LE TRAITEMENT DES AFFECTIONS PULMONAIRES D'ENFANTS PRÉMATURÉS

(30) Priorität: 12.11.2008 DE 102008056875
(43) Veröffentlichungstag der Anmeldung: 17.08.2011
(73) Patentinhaber: Rupprecht, Sabine, 91080 Uttenreuth (DE)
(72) Erfinder: Rupprecht, Sabine, 91080 Uttenreuth (DE)
(74) Vertreter: Hofstetter, Schurack & Partner
(86) Internationale Anmeldenummer: PCT/EP2009/065026
(87) Internationale Veröffentlichungsnummer: WO 2010/055081

(56) Entgegenhaltungen:
- KINSELLA J P ET AL: "Bronchopulmonary dysplasia" LANCET THE, LANCET LIMITED. LONDON, GB, Bd. 367, Nr. 9520, 29. April 2006 (2006-04-29), Seiten 1421-1431, XP025094032 ISSN: 0140-6736 [gefunden am 2006-04-29]
- FUNK AMY J ET AL: "Effects of leukotriene inhibition on pulmonary morphology in rat pup lungs exposed to hyperoxia" COMPARATIVE MEDICINE (MEMPHIS), Bd. 57, Nr. 2, April 2007 (2007-04), Seiten 186-192, XP002567385 ISSN: 1532-0820
- DEMIR KORCAN ET AL: "Clarithromycin, montelukast, and pentoxifylline combination treatment ameliorates experimental neonatal hyperoxic lung injury." THE JOURNAL OF MATERNAL-FETAL & NEONATAL MEDICINE : THE OFFICIAL JOURNAL OF THE EUROPEAN ASSOCIATION OF PERINATAL MEDICINE, THE FEDERATION OF ASIA AND OCEANIA PERINATAL SOCIETIES, THE INTERNATIONAL SOCIETY OF PERINATAL OBSTETRICIANS JUN 2008, Bd. 21, Nr. 6, Juni 2008 (2008-06), Seiten 407-413, XP009129123 ISSN: 1476-4954
- ANONYMOUS: "Montelukast in very low birthweight infants"[Online] 9. Mai 2008 (2008-05-09), XP002567386 Gefunden im Internet: URL:http://clinicaltrials.gov/ct2/show/NCT 00492102> [gefunden am 2010-02-03]
- COOK A J ET AL: "Cysteinyl leukotriene involvement in chronic lung disease in premature infants" EUROPEAN RESPIRATORY JOURNAL, Bd. 9, Nr. 9, 1996, Seiten 1907-1912, XP002567387 ISSN: 0903-1936
- GRONEFCK PETER ET AL: "Association of pulmonary inflammation and increased microvascular permeability during the development of bronchopulmonary dysplasia: A sequential analysis of inflammatory mediators in respiratory fluids of high-risk preterm neonates" PEDIATRICS, Bd. 93, Nr. 5, 1994, Seiten 712-718, XP009129142 ISSN: 0031-4005
- MIRRO R ET AL: "INCREASED AIRWAY LEUKOTRIENE LEVELS IN INFANTS WITH SEVERE BRONCHOPULMONARY DYSPLASIA" AMERICAN JOURNAL OF DISEASES OF CHILDREN, Bd. 144, Nr. 2, 1990, Seiten 160-161, XP009129141 ISSN: 0002-922X
- STENMARK K R ET AL: "RECOVERY OF PLATELET ACTIVATING FACTOR AND LEUKOTRIENES FROM INFANTS WITH SEVERE BRONCHOPULMONARY DYSPLASIA CLINICAL IMPROVEMENT WITH CROMOLYN TREATMENT", THE AMERICAN REVIEW OF RESPIRATORY DISEASE, AMERICAN THORACIC SOCIETY, US, vol. 131, no. 4, Suppl, 1 January 1985 (1985-01-01), page A236, XP009179825, ISSN: 0003-0805

## Beschreibung

Die Erfindung betrifft Behandlungsmöglichkeiten von Lungenerkrankungen bei frühgeborenen Kindern.

Aufgrund der technischen Fortschritte der Medizin in den letzten Jahrzehnten gibt es mehr und mehr lebend zur Welt kommende Frühgeborene, welche nicht zuletzt aufgrund ihrer Frühgeburtlichkeit dem Risiko der Entwicklung einer Lungenerkrankung unterliegen. Insbesondere die sogenannte bronchopulmonale Dysplasie (BPD) tritt bei frühgeborenen Kindern vergleichsweise häufig auf, da diese Kinder in der Regel über einen längeren Zeitraum künstlich beatmet werden müssen, um zum Beispiel das Neugeborenen-Atemnotsyndrom zu behandeln. Die Inzidenz der BPD bleibt aufgrund dieser Tatsache weiterhin hoch und steigt sogar weiter an. In Deutschland erkranken beispielsweise jährlich ungefähr 1300 bis 1500 Neugeborene an einer BPD, wobei deren Inzidenz bei 1 bis 1,8 Fällen pro 1000 Lebendgeborenen liegt und eine der häufigsten chronischen Lungenerkrankungen des Kindesalters darstellt. Die BPD ist mit einer Reihe von Komplikationen assoziiert und geht mit einer erhöhten Mortalitätsrate einher. Zu den häufigsten akuten Komplikationen zählen:
1. wochen- bis monatelange Sauerstoff- und Beatmungsabhängigkeit durch verminderte Oxygenierungs-Funktion der Lunge;
2. gehäuftes Auftreten von pulmonalen und systemischen Infektionen, die wahrscheinlich durch Schleimverhalt in schlecht belüfteten Lungenabschnitten verursacht werden;
3. Pneumothorax und Lungenemphysem durch Überdehnung einzelner Areale unter Beatmung; und
4. pulmonale Hypertonie durch Gefäßkonstriktion in den schlecht belüfteten Lungenabschnitten, wodurch in der Folge Cor pulmonale mit folgendem Herzversagen entstehen kann.

Zu den Langzeitfolgen zählen Dystrophie, psychomotorische Entwicklungsstörungen sowie Rehospitalisierungen aufgrund rezidivierender pulmonaler Infektionen vor allem im ersten Lebensjahr. Besonders problematisch ist, dass bei vielen Kindern die Erkrankung selbst dann weiter fortschreitet, wenn die auslösenden Faktoren beseitigt wurden, und letztlich zu einer völligen Zerstörung der Lunge mit Todesfolge führen kann.

Das bisherige Therapiekonzept der BPD berücksichtigt die bereits bekannten pathophysiologischen Ursachen dieser Erkrankung und beruht auf verschiedenen therapeutischen Grundprinzipien. Diese beinhalten die pränatale Prävention mit Glukokortikoiden zur Unterstützung der Lungenreifung und erstrecken sich dann bei einer manifesten BPD von Sauerstoffapplikation über Flüssigkeitsrestriktion, ggf. verbunden mit dem Einsatz von Diuretika. Weiterhin sind Inhalationstherapien sowie eine erneute systemische Gabe von Glukokortikoiden als mögliches Behandlungsschema vorgesehen. Ziel der Behandlungen ist dabei stets die Vermeidung eines Barotraumas der Lunge durch möglichst schonende Beatmung und frühzeitiges Ausweichen auf eine sogenannte continuous positive airway pressure-Beatmung (CPAP-Beatmung) sowie die großzügige Indikationsstellung zur Surfactantsubstitution.

Trotz dieses umfangreichen Therapiekonzepts liegt die Mortalität der BPD aktuell bei ca. 5-10%. Die häufigsten Todesursachen im ersten Lebensjahr betreffen kardiopulmonale Komplikationen, pulmonale Infektionen, Sepsis oder den plötzlichen Kindstod. In der Pathogenese der BPD gelten bereits einige Faktoren als bekannt und erforscht, etliche pathogenetische Mechanismen sind jedoch bislang trotz weltweit intensiver Forschungstätigkeit weiterhin ungeklärt. Bekannt ist beispielsweise, dass perinatale Entzündungsprozesse in der unreifen Lunge eine erhöhte Gefäßpermeabilität bewirken. Aus diesem Grund werden derzeit Corticosteroide aufgrund ihrer antiinflammatorischen Wirkung hochdosiert eingesetzt. Weitere Gründe für das Entstehen der BPD liegen in einer anfänglichen Wassereinlagerung in der unreifen Lunge sowie in den chemischen und mechanischen Belastungen durch die Beatmung mit hohen Sauerstoffgehalten. Zusätzlich können biologische Schädigungen durch mikrobielle Erreger auftreten. Betroffen sind die Lungenbläschen, die Atemwege und die Blutgefäße der Lunge, die sich verengen und damit zu einem erhöhten Druck im Lungenkreislauf und einer Belastung für die rechte Herzkammer führen können.

Es hat sich jedoch herausgestellt, dass durch die Gabe von Steroiden schweren Beeinträchtigungen der cerebralen Entwicklung dieser Kinder als Nebenwirkung auftreten können. Neben einer schweren Schädigung der Lunge treten bei bis zu 50% der betroffenen Patienten in der Regel hochgradige geistige Behinderungen auf.

Die Publikation KINSELLA, J. P. et al: "Bronchopulmonary dysplasia" LANCET THE, LANCET LIMITED. LONDON, GB, Bd. 367, Nr. 9520, 29. April 2006 (2006-04-29), Seiten 1421-1431, beschreibt verschiedene Therapiekonzepte der bronchopulmonalen Dysplasie (BPD) für frühgeborene Kinder.

In der Publikation FUNK, AMY J. et al: "Effects of leukotriene inhibition on pulmonary morphology in rat pup lungs exposed to hyperoxia" COMPARATIVE MEDICINE (MEMPHIS), Bd. 57, Nr. 2, April 2007 (2007-04), Seiten 186-192, wird eine Studie an insgesamt 39 Ratten beschrieben. Den Ratten wurde dabei unter anderem Zileuton in einer Dosierung von 20 mg pro Kilogramm Körpergewicht und Zafirlukast in einer Dosis von 40 mg pro Kilogramm Körpergewicht über einen Zeitraum von sieben Tagen verabreicht. Dabei wurden keine signifikanten Unterschiede zwischen der Gruppe, die mit Zafirlukast behandelt wurde, und einer Kontrollgruppe, die mit Salzlösung behandelt wurde, festgestellt.

Die Publikation DEMIR, KORCAN et al: "Clarithromycin, montelukast, and pentoxifylline combination treatment ameliorates experimental neonatal hyperoxic lung injury." THE JOURNAL OF MATERNAL-FETAL & NEONATAL MEDICINE : THE OFFICIAL JOURNAL OF THE EUROPEAN ASSOCIATION OF PERINATAL MEDICINE, THE FEDERATION OF ASIA AND OCEANIA PERINATAL SOCIETIES, THE INTERNATIONAL SOCIETY OF PERINATAL OBSTETRICIANS JUN 2008, Bd. 21, Nr. 6, Juni 2008 (2008-06), Seiten 407-413, beschreibt ebenfalls verschiedene Experimente, die an insgesamt 47 neugeborenen Ratten durchgeführt wurden.

Die Internet-Publikation "Montelukast in very low birthweight infants" (http://clinicaltrials.gov/ct2/show/NCT00492102) betrifft eine Studie, in welcher die Pharmakokinetik von Montelukast bei Frühgeborenen mit geringem Geburtsgewicht untersucht werden soll.

Die Publikation STENMARK K.R. et al: "Recovery of Platelet Activating Factor and leukotrienes from infants with severe bronchopulmonary dysplasia: clinical improvement with Cromolyn treatment", AMERICAN REVIEW OF RESPIRATORY DISEASE, Bd. 131, Nr. 4, Suppl, 1985, Seite A236, beschreibt die Verwendung von Cromolyn in 10 Kindern mit bronchopulmonaler Dysplasie. Aufgabe der vorliegenden Erfindung ist es, verbesserte Behandlungsmöglichkeiten für frühgeborene Kinder bereitzustellen, mit deren Hilfe der oft lebensentscheidende Erkrankungsverlauf vermeidbar oder zumindest vorteilhaft beeinflussbar ist.

Die Aufgabe wird erfindungsgemäß durch eine Verwendung von Montelukast und/oder Zafirlukast und/oder Pranlukast zur Herstellung einer pharmazeutischen Zusammensetzung zur Prophylaxe und/oder Behandlung von bronchopulmonaler Dysplasie bei frühgeborenen Kindern sowie durch eine pharmazeutische Zusammensetzung gemäß Anspruch 3 zur Prophylaxe und/oder Behandlung von bronchopulmonaler Dysplasie bei einem frühgeborenen Kind gelöst. Vorteilhafte Ausgestaltungen mit zweckmäßigen Weiterbildungen der Erfindung sind in den jeweiligen Unteransprüchen angegeben, wobei vorteilhafte Ausgestaltungen der Verwendung als vorteilhafte Ausgestaltungen der pharmazeutische Zusammensetzung und umgekehrt anzusehen sind.

Erfindungsgemäß ist eine Verwendung von Montelukast und/oder Zafirlukast und/oder Pranlukast zur Herstellung einer pharmazeutischen Zusammensetzung zur Prophylaxe und/oder Behandlung von bronchopulmonaler Dysplasie bei frühgeborenen Kindern vorgesehen. Es hat sich herausgestellt, dass der Erkrankungsverlauf bei Lungenerkrankungen von frühgeborenen Kindern mit einer starken entzündlichen Reaktion im Bereich des alveolären Epithels einhergeht. Dabei wurde festgestellt, dass erhöhte intrapulmonale Spiegel an Interleukinen und anderen Akut-Phaseproteinen auftreten. Leukotriene sind Entzündungsmediatoren, die im Zusammenhang mit Entzündungsreaktionen bei Asthma bronchiale bekannt sind. Insbesondere Cysteinylleukotriene sind dabei offenbar verantwortlich für das Entstehen von Entzündungen, welche wiederum verengend auf die Atemwege der frühgeborenen Kinder wirken und deren Lungenfunktion schwächen. Im Unterschied zum äußerst geringen Behandlungserfolg bei Asthma bronchiale hat sich überraschenderweise herausgestellt, dass die in den Ansprüchen genannten Leukotrien-Inhibitoren sowohl zur präventiven als auch zur kurativen Behandlung von bei frühgeborenen Kindern auftretender bronchopulmonaler Dysplasie mit großem Behandlungserfolg eingesetzt werden können, um durch Blockierung des Leukotrien-Systems eine effektive antiinflammatorische Wirkung zu entfalten und eine vermehrte Schleimsekretion in der Lunge zu verhindern. Den Grund für diese überraschende Wirksamkeit vermutet die Anmelderin - ohne auf diese Ansicht festgelegt zu werden - in den abweichenden Ursachen und den unterschiedlichen Manfestierungsformen gegenüber Asthma bronchiale. Weiterhin hat sich herausgestellt, dass mit Hilfe dieser Leukotrien-Inhibitoren ein Gewebeumbau der Lunge durch Fibroblastenvermehrung und eine Zerstörung der Extrazellularmatrix bei Frühgeborenen zuverlässig verhindert werden. Montelukast und/oder Zafirlukast und/oder Pranlukast kann dabei grundsätzlich in jeder pharmakologisch wirksamen Form verwendet werden. Beispielsweise kann es als Reinsubstanz,
als Enantiomer, als Diastereomere, als racemisches Gemisch, als pharmakologisch akzeptables Salz, solvatisiert und/oder unsolvatisiert verwendet werden. Zusammenfassend ermöglicht die erfindungsgemäße Verwendung somit eine wesentlich verbesserte Behandlungsmöglichkeit für frühgeborene Kinder, wobei im Vergleich zu den bisherigen Therapieansätzen wesentlich geringere und seltenere Nebenwirkungen auftreten. Der oft lebensentscheidende Erkrankungsverlauf ist somit von vornherein vermeidbar oder kann zumindest erheblich abgeschwächt werden.

Montelukast und/oder Zafirlukast und/oder Pranlukast werden als Leukotrien-Rezeptor-Antagonist verwendet. Leukotrien-Rezeptor-Antagonisten binden an Leukotrien-Rezeptoren (LTD4-Rezeptoren) und blockieren dadurch die Wirkung der Leukotriene im Entzündungsprozess. Hierdurch wird eine antiinflammatorische Wirkung im Bronchialsystem des frühgeborenen Kinds erzielt.

Montelukast, Zafirlukast und Pranlukast greifen direkt in inflammatorische Prozesse ein, indem sie mit einer hohen Affinität und Selektivität an die in den Atemwegen vorhandenen Cysteinyl-Leukotrien-Rezeptoren binden und durch deren Blockade die proinflammatorische Wirkung von Leukotrienen hemmen.

Weitere Vorteile ergeben sich, indem die pharmazeutische Zusammensetzung zur oralen und/oder intravenösen und/oder aerogenen und/oder rektalen Verabreichung hergerichtet wird. Hierdurch kann die pharmazeutische Zusammensetzung besonders flexibel verabreicht werden, so dass der Entwicklungsgrad des betreffenden Frühgeborenen optimal berücksichtig werden kann.

Ein weiterer Aspekt der Erfindung betrifft eine pharmazeutische Zusammensetzung zur Anwendung in der Prophylaxe und/oder Behandlung von bronchopulmonaler Dysplasie bei frühgeborenen Kindern, wobei die pharmazeutische Zusammensetzung Montelukast und/oder Zafirlukast und/oder Pranlukast umfasst. Hierdurch wird eine verbesserte Behandlungsmöglichkeit für frühgeborene Kinder ermöglicht, mit deren Hilfe der oft lebensentscheidende Erkrankungsverlauf vermeidbar oder zumindest vorteilhaft beeinflussbar ist. Die Vorteile einer derartigen pharmazeutischen Zusammensetzung sind den vorhergehenden Ausführungen zur erfindungsgemäßen Verwendung zu entnehmen. Die im Zusammenhang mit der erfindungsgemäßen Verwendung vorgestellten bevorzugten Ausführungsformen und Weiterbildungen sowie deren Vorteile gelten entsprechend für das erfindungsgemäße Verfahren und umgekehrt.

Indem die pharmazeutische Zusammensetzung einen Leukotrien-Rezeptor-Antagonisten umfasst, kann die Entstehung von Leukotrienen vorteilhaft blockiert werden. Leukotrien-Rezeptor-Antagonisten binden an Leukotrien-Rezeptoren (LTD4-Rezeptoren) und blockieren dadurch die Wirkung der Leukotriene im Entzündungsprozess. Hierdurch wird eine antiinflammatorische Wirkung im Bronchialsystem des frühgeborenen Kinds erzielt.

Montelukast, Zafirlukast und Pranlukast greifen direkt in inflammatorische Prozesse ein, indem sie mit einer hohen Affinität und Selektivität an die in den Atemwegen vorhandenen Cysteinyl-Leukotrien-Rezeptoren binden und durch deren Blockade die proinflammatorische Wirkung von Leukotrienen hemmen.

In weiterer Ausgestaltung der Erfindung hat es sich als vorteilhaft gezeigt, wenn die pharmazeutische Zusammensetzung zur Verabreichung von Montelukast und/oder Zafirlukast und/oder Pranlukast in einer Dosierung zwischen (0,2± 10%) mg/kg/d und (3,5± 10%) mg/kg/d, insbesondere zwischen (1,0± 10%) mg/kg/d und (2,0± 10%) mg/kg/d, hergerichtet ist. Die Bezeichnung mg/kg/d steht dabei in der gesamten vorliegenden Offenbarung für die zu verabreichende Menge an Wirksubstanz in Milligramm pro Kilogramm Körpergewicht des Patienten und pro Tag (d). Da Frühgeborene in der Regel ein Geburtsgewicht unter 1500 g und teilweise von 500 g oder weniger besitzen, wird durch den genannten Dosierungsbereich eine hohe Wirksamkeit der pharmazeutischen Zusammensetzung bei gleichzeitig geringstmöglichen Nebenwirkungen sichergestellt. Die Herrichtung der pharmazeutischen Zusammensetzung kann dabei grundsätzlich in Abhängigkeit der gewünschten Verabreichungsform und -häufigkeit gewählt sein.

Indem die pharmazeutische Zusammensetzung eine geeignete Menge wenigstens eines pharmazeutisch akzeptablen Trägerstoffs umfasst, kann eine verbesserte Dosierbarkeit sowie eine vereinfachte Handhabung der pharmazeutischen Zusammensetzung erzielt werden. Bei dem Trägerstoff handelt es sich vorzugsweise um einen inerten Trägerstoff, so dass ein unerwünschter Abbau der pharmazeutisch wirksamen Inhaltsstoffe der pharmazeutischen Zusammensetzung zuverlässig ausgeschlossen wird. Pharmazeutisch akzeptable Träger, die zur Verwendung in diesen Zusammensetzungen geeignet sind, können beispielsweise ein oder mehrere Bindemittel umfassen, die ausgewählt sind aus einer Gruppe, die inerte Bindemittel umfassen, wobei grundsätzlich reaktive Bindemittel wie Lactose oder andere Mono- oder Disaccharide vorgesehen sein können.

Weiterhin wird ein nicht zur Erfindung gehörendes Verfahren zum Behandeln und/oder zur Prophylaxe einer Lungenerkrankung, insbesondere einer bronchopulmonalen Dysplasie, bei einem frühgeborenen Kind offenbart, bei welchem dem frühgeborenen Kind eine pharmakologisch wirksame Menge wenigstens eines Leukotrien-Inhibitors verabreicht wird. Hierdurch wird eine verbesserte Behandlung für frühgeborene Kinder erzielt, mit deren Hilfe der oft lebensentscheidende Erkrankungsverlauf vermeidbar oder zumindest vorteilhaft beeinflussbar ist. Die Vorteile eines derartigen Verfahrens sind den vorhergehenden Ausführungen zur erfindungsgemäßen Verwendung sowie zur erfindungsgemäßen pharmazeutischen Zusammensetzung zu entnehmen.

Es kann vorgesehen sein, dass dem frühgeborenen Kind wenigstens ein Leukotrien-Rezeptor-Antagonist und/oder wenigstens ein Leukotrien-Synthese-Antagonist als Leukotrien-Inhibitor verabreicht wird. Leukotrien-Rezeptor-Antagonisten binden an Leukotrien-Rezeptoren (LTD4-Rezeptoren) und blockieren dadurch die Wirkung der Leukotriene im Entzündungsprozess. Hierdurch wird eine antiinflammatorische Wirkung im Bronchialsystem des frühgeborenen Kinds erzielt. Alternativ oder zusätzlich kann als Leukotrien-Inhibitor auch ein Leukotrien-Synthese-Antagonist verwendet werden, dessen pharmakologische Wirkung jedoch in der Hemmung der Leukotriensynthese selbst besteht. Somit kann die Entstehung von Leukotrienen vorteilhaft über unterschiedliche Mechanismen blockiert werden.

Es kann weiterhin vorgesehen sein, dass dem frühgeborenen Kind Montelukast und/oder Zafirlukast und/oder Pranlukast als Leukotrien-Rezeptor-Antagonist und/oder Zileuton als Leukotrien-Synthese-Antagonist verabreicht wird. Montelukast, Zafirlukast und Pranlukast greifen direkt in inflammatorische Prozesse ein, indem sie mit einer hohen Affinität und Selektivität an die in den Atemwegen vorhandenen Cysteinyl-Leukotrien-Rezeptoren binden und durch deren Blockade die proinflammatorische Wirkung von Leukotrienen hemmen. Alternativ oder zusätzlich kann das antineoplastische Hydroxyurea-Derivat Zileuton (Zyflo®) als Leukotrien-Synthese-Antagonist verwendet werden, welches durch Hemmung der 5-Lipoxygenase die Synthese von Leukotrienen aus Arachidonsäure blockiert. Es ist jedoch zu betonen, dass grundsätzlich auch andere als die genannten Leukotrien-Rezeptor-Antagonisten bzw. als der genannte Leukotrien-Synthese-Antagonist vorgesehen sein können.

Es kann weiterhin vorgesehen sein, dass dem frühgeborenen Kind eine erhöhte Energiegabe zugeführt wird und/oder wenigstens ein weiterer Wirkstoff aus der Gruppe der Entzündungshemmer, Antibiotika, Anitasthmatika, Bronchodilatatoren, Betasympathomimetica, Diuretika, Parasympatholytica, Vitamine, Cytochrom P450-Induktoren und/oder Vasodilatanzien verabreicht wird. Aufgrund des erhöhten Energiebedarfs der erkrankten Frühgeborenen hat sich eine erhöhte Energiegabe durch hochkalorische Ernährung als vorteilhaft gezeigt. Alternativ oder zusätzlich können mit Hilfe des weiteren Wirkstoffs insbesondere die vorstehend unter 1. bis 4. genannten häufigsten akuten Komplikationen gezielt behandelt werden. Die Verwendung von Antibiotika ermöglicht beispielsweise die Unterbindung bakterieller Infektionen. Mit Hilfe von Diuretika kann die Gefahr der Entstehung eines Lungenödems vorteilhaft verringert werden.

Es kann weiterhin vorgesehen sein, dass dem frühgeborenen Kind Clarithromycin, Pentoxifyllin, Vitamin A, Provitamin A, Hydrochlorothiazid, Spironolacton, Furosemid, Salbutamol, Ipratropiumbromid, Phenobarbital, Phenytoin, Rifampicin und/oder wenigstens ein Steroid, insbesondere ein Corticosteroid und/oder ein Glucocorticoid wie Dexamethason, als weiterer Wirkstoff verabreicht wird. Beispielsweise kann mit Hilfe von Steroiden wie Dexamethason eine schnelle Verbesserung der Lungenfunktion und eine Verringerung des Auftretens von BPD erzielt werden, wobei im Unterschied zu den bekannten Therapieansätzen aufgrund der synergistischen Wirkung mit dem Leuktorien-Inhibitor wesentlich geringere Dosierungen notwendig sind, um vergleichbare Wirkungen zu erzielen. Dementsprechend wird jedoch die Häufigkeit von Nebenwirkungen vorteilhaft verringert.

Es kann weiterhin vorgesehen sein, dass eine verabreichte Dosis zumindest des Leukotrien-Inhibitors gegenüber einer Dosis bei einer Verabreichung ohne Cytochrom P450-Induktor erhöht wird, wenn als weiterer Wirkstoff ein Cytochrom P450-Induktor vorgesehen ist. Da Leukotrien-Inhibitoren - beispielsweise Montelukast - über das Cytochrom P450-System (CYP3A4) metabolisiert werden, können Wirkstoffe, die CYP3A4 induzieren (z.B. Phenobarbital, Phenytoin oder Rifampicin) die Plasmakonzentration und somit die Wirkung von Montelukast erniedrigen. Um diesen Effekt zu neutralisieren hat es sich als vorteilhaft gezeigt, die Dosis des Leukotrien-Inhibitors zumindest derart zu erhöhen, dass die erhöhte Abbaurate kompensiert wird und sich nach der Verabreichung eine Plasmakonzentration ergibt, die mit derjenigen einer Verabreichung ohne Cytochrom P450-Induktor korrespondiert. Zu diesem Zweck kann beispielsweise eine den Leukotrien-Inhibitor umfassende pharmazeutische Zusammensetzung entsprechend mit einer erhöhten Konzentration bzw. Menge an Leukotrien-Inhibitor hergerichtet sein. Alternativ oder zusätzlich kann der Leukotrien-Inhibitor in entsprechender Dosis getrennt vom Cytochrom P450-Induktor verabreicht werden. Analog kann natürlich auch die Dosis weiterer, vom erhöhten Abbau betroffener Wirkstoffe entsprechend erhöht werden.

Ein besonders flexibles Prophylaxe- und/oder Behandlungsverfahren kann dadurch ermöglicht werden, dass der wenigstens eine Leukotrien-Inhibitor und der wenigstens eine weitere Wirkstoff gemeinsam und/oder zeitlich getrennt voneinander verabreicht werden.

Es kann weiterhin vorgesehen sein, dass dem frühgeborenen Kind zumindest der wenigstens eine Leukotrien-Inhibitor einmal, zweimal und/oder mehrmals täglich über einen geeigneten Zeitraum intermittierend verabreicht wird und/oder bei welchem dem frühgeborenen Kind zumindest der wenigstens eine Leukotrien-Inhibitor durchgehend über einen geeigneten Zeitraum verabreicht wird. Beispielsweise kann der wenigstens eine Leukotrien-Inhibitor ein-, zwei-, drei-, vier-, fünf-, sechsmal oder häufiger in täglichen Einzeldosierungen oder durchgehend, beispielsweise über eine Dauertropfinfusion, verabreicht werden. Auf diese Weise kann die Plasmakonzentration bzw. der zeitliche Verlauf der Plasmakonzentration gezielt an das jeweils vorliegende Krankheitsbild angepasst werden.

Wenn dem frühgeborenen Kind zumindest der wenigstens eine Leukotrien-Inhibitor oral und/oder intravenös und/oder aerogen und/oder rektal verabreicht wird, ist eine besonders flexible Möglichkeit zur Verabreichung des pharmazeutisch aktiven Wirkstoffs gegeben.

Es kann weiterhin vorgesehen sein, dass der wenigstens eine Leukotrien-Inhibitor dem frühgeborenen Kind in einer Dosierung zwischen (0,2± 10%) mg/kg/d und (3,5± 10%) mg/kg/d, insbesondere zwischen (1,0± 10%) mg/kg/d und (2,0± 10%) mg/kg/d, verabreicht wird. Da Frühgeborene in der Regel ein Geburtsgewicht unter 1500 g und teilweise von 500 g oder weniger besitzen, wird durch den genannten Dosierungsbereich eine hohe Wirksamkeit der pharmazeutischen Zusammensetzung bei gleichzeitig geringstmöglichen Nebenwirkungen sichergestellt. Vorzugsweise wird dem Frühgeborenen eine in Abhängigkeit der gewünschten Verabreichungsform und -häufigkeit entsprechend hergerichtete pharmazeutische Zusammensetzung verabreicht.

Eine weitere Verbesserung des Prophylaxe- und/oder Behandlungsverfahrens kann dadurch ermöglicht werden, dass der wenigstens eine Leukotrien-Inhibitor dem frühgeborenen Kind in der ersten Behandlungswoche in einer Dosis von (1,0 ± 10%) mg/kg/d, in der zweiten Behandlungswoche in einer Dosis von (1,5 ± 10%) mg/kg/d und in der dritten Behandlungswoche in einer Dosis von (2,0 ± 10%) mg/kg/d verabreicht wird. Hierdurch wird eine besonders hohe Verträglichkeit und Nebenwirkungsarmut sichergestellt, da einerseits bereits bei niedrigen Dosierungen eine vorteilhafte therapeutische Wirkung sichergestellt ist und andererseits die Dosissteigerung beim Auftreten von dosisabhängigen Unverträglichkeiten abgebrochen und die Behandlung bzw. Prophylaxe mit einer niedrigeren Dosierung fortgesetzt werden kann.

Es kann weiterhin vorgesehen sein, dass der wenigstens eine Leukotrien-Inhibitor über einen Zeitraum von etwa acht Wochen verabreicht wird, wenn das frühgeborene Kind unter einer moderaten Lungenerkrankung leidet, oder dass der wenigstens eine Leukotrien-Inhibitor über einen Zeitraum von etwa vierundzwanzig Wochen verabreicht wird, wenn das frühgeborene Kind unter einer schweren Lungenerkrankung leidet. Unter einer Zeitdauer von etwa acht Wochen sind dabei zwischen sieben und neun Wochen, unter einer Zeitdauer von etwa vierundzwanzig Wochen sind zwischen dreiundzwanzig und fünfundzwanzig Wochen zu verstehen, wobei darüber hinaus zusätzliche Abweichungen von bis zu fünf Tagen vorgesehen sein können. Als klinische Symptome für die Schwere der Erkrankung können beispielsweise erhöhte Atemfrequenz, vertiefte angestrengte Atmung mit Einziehungen am Brustkorb, vermehrtes Bronchialsekret, Husten, Wachstumsverzögerung und livide Haut- und Schleimhaut herangezogen werden. Starke Lungenerkrankungen können dadurch identifiziert werden, dass sich im Röntgenbild der Lunge u.a. diffuse Überblähungsbezirke neben unzureichend belüfteten Bereichen (Atelektasen) finden. Diagnose und Einteilung der Schwere der Lungenerkrankung können auch anhand des zu einer ausreichenden Sauerstoffsättigung des Blutes notwendigen Sauerstoffbedarfs zu einem festgelegten Alter des Kindes erfolgen. Definitionsgemäß ist dabei der Sauerstoffbedarf zum Zeitpunkt eines korrigierten Alters von 36 Schwangerschaftswochen (SSW) maßgebend. Man unterscheidet zwischen einer milden Lungenerkrankung (mit 36 SSW kein erhöhter Sauerstoffbedarf mehr), einer moderaten Lungenerkrankung (weniger als 30% Sauerstoff in der Einatemluft notwendig) und einer schweren Lungenerkrankung (mehr als 30% und/oder Beatmung bzw. Atemunterstützung notwendig). Der Schweregrad der Lungenerkrankung wird üblicherweise anhand des sogenannten NICHD-Scores (milde, moderate, schwere BPD) festgestellt. Für die Entscheidungsphase, welcher Schweregrad vorliegt, kann pro Patient ein Zeitraum von bis zu vier Wochen vorgesehen werden. Zeichnet sich ab, dass das Frühgeborene nur einer milden BPD unterliegt, kann vorgesehen sein, dass von einer Verabreichung zumindest des Leukotrien-Inhibitors abgesehen wird.

Weitere Merkmale der Erfindung ergeben sich aus den Ansprüchen und dem Ausführungsbeispiel.

In der Beschreibung des Ausführungsbeispiels werden als Abkürzungen:
BPD: Bronchopulmonale Dysplasie
CPAP: continuous positive airway pressure
d: Tag
FiO₂: inspiratorische Sauerstofffraktion
FG: Frühgeborenes Kind
kg : Kilogramm
mg: Milligramm
NICHD: National Institute of Child Health and Human Development
PMA: postmenstrual age
SSW: Schwangerschaftswoche
verwendet.

Im Rahmen des vorliegend beschriebenen Heilversuchs wurden im Rahmen eines standardisierten Projekts in mehreren neonatologischen Abteilungen Daten gewonnen, aus denen hervorgeht, dass durch die Behandlung mit einem Leukotrien-Inhibitor im Vergleich zu bisherigen Therapieformen eine weniger eingreifende und weniger nebenwirkungsreiche antiinflammatorische Therapie bei diesen Patienten zu erreichen ist.

In der ersten Phase wurde der Schweregrad der Lungenerkrankung von mehreren Frühgeborenen festgestellt. Bei der Lungenerkrankung handelte es sich um bronchopulmonale Dysplasie, die anhand des NICHD-Scores (milde, moderate, schwere BPD) kategorisiert wurde. Zur Beurteilung des Schweregrades der bronchopulmonalen Dysplasie kann unter anderem auch der Pulmonary a-cuity score (PAS) erhoben werden. Der PAS stellt einen pulmonalen Score zur Bewertung des Schweregrades einer bronchopulmonalen Dysplasie dar und ist definiert als die inspiratorische Sauerstofffraktion (FiO₂) multipliziert mit dem Support Score, addiert mit dem Medical Score. Der PAS schließt folgende Parameter ein: FiO₂, Beatmungsform sowie konventionelle Medikation zur Behandlung einer bronchopulmonalen Dysplasie. Dieser an sich bekannte Score bewegt sich in einem Bereich von 0,21 bis 2,95. Je kleiner der PAS-Wert ist, desto besser wird das pulmonale Outcome eingeschätzt.

Für die Feststellung des Schweregrades wurde pro Patient ein Zeitraum von vier Wochen anberaumt. Zeichnete sich ab, dass das Frühgeborene nur einer milden BPD unterliegt, wurde dieser Patientengruppe die pharmazeutische Zusammensetzung mit dem Leukotrien-Inhibitor nicht angeboten.

Patienten mit moderater BPD wurde ein Leukotrien-Inhibitor über acht Wochen verabreicht. Die anschließend erhobenen klinischen und laborchemischen Parameter konnten - soweit vorhanden - zu Vergleichszwecken verwendet werden. Patienten mit schwerer BPD und somit infauster Prognose wurde die pharmazeutische Zusammensetzung mit dem Leukotrien-Inhibitor über einen längeren Zeitraum von sechs Monaten zugeführt. Dabei konnten bei keinem der bislang behandelten Patienten Nebenwirkungen beobachtet werden.

Eine Randomisierung dieses Versuches erfolgte in der Weise, dass die pharmazeutische Zusammensetzung mit dem Leukotrien-Inhibitor allen kritisch kranken Patienten angeboten und die Gruppe mit fehlender Einwilligung der Erziehungsberechtigten als klinische Kontrollgruppe verwendet wurde. Es wurden bei allen Patienten die routinemäßig in der Neonatologie üblicherweise erfassten Parameter in regelmäßigen Abständen in gesonderte Dokumentationsbögen übertragen und einer Auswertung unterzogen. Im Anschluss an die 24 ersten Therapiewochen wurde eine weitere 24 Wochen lang dauernde Nachbeobachtungsphase durchgeführt.

Die pharmazeutische Zusammensetzung mit dem Leukotrien-Inhibitor wurde gemäß folgendem Dosisschema verabreicht:

Wenn keine unerwünschten Nebenwirkungen auftraten, wurde die Dosierung des Leukotrien-Inhibitors bei jedem Patienten ausgehend von einer Startdosis von etwa 1,0 mg/kg Körpergewicht/Tag (d) nach einer Woche um 0,5 mg/kg Körpergewicht/d auf 1,5 mg/kg Körpergewicht/d gesteigert. Nach einer weiteren Woche wurde die Dosis dann erneut um 0,5 mg/kg Körpergewicht/d auf eine Maximaldosis von etwa 2,0 mg/kg Körpergewicht/d erhöht. Als Leukotrien-Inhibitoren waren Montelukast, Zafirlukast und Pranlukast einzeln und in beliebigen Kombinationen vorgesehen. Es erfolgte vorab eine Selektionierung derjenigen Patienten, die zu einem Hochrisikokollektiv zur Entwicklung einer bronchopulmonalen Dysplasie gehörten. Zu diesem Hochrisikokollektiv zählen Patienten:
- mit einem Geburtsdatum ≤ 32. Schwangerschaftswoche;
- mit einem Geburtsgewicht ≤ 1500 g;
- mit einem Gewicht unter der 3. Gewichtsperzentile (VLBW, very low birth weight) ohne initialem Vorliegen eines RDS (respiratory distress syndrom);
- mit einer progredienten respiratorischen Insuffizienz ab der 2. Lebenswoche;
- welche folgenden Anamnesekriterien aufweisen: systemische Infektion, persistierender Ductus arteriosus, Kolonisation mit spezifischen Mikroorganismen (Ureaplasma, CMV); und/oder
- welche im Röntgen-Thorax (ab der zweiten Lebenswoche) eine milchige Trübung des Lungenparenchyms aufweisen.

Für die Therapie mit einem Leukotrien-Inhibitor wurden nach dem Screening der oben genannten Risikofaktoren nur Patienten berücksichtigt:
- welche vor/in der 32. Schwangerschaftswoche geboren wurden;
- welche > 28. Lebenstag noch immer einen FiO₂ >0,21 benötigen; und
- welche ≥ 36. Woche postmenstrual age (PMA) eine FiO₂ >0,21 benötigen.

Als Ausschlusskriterien für den Heilversuch wurden festgelegt:
- Blutgerinnungsstörung;
- Vorliegen von Neugeborenen-Krampfanfällen;
- Vorliegen erhöhter Serumtransaminasen;
- Vorliegen von Hauterkrankungen; und
- Therapie mit Phenobarbital.

Da es sich bei Phenobarbital um einen Cytochrom P450-Induktor handelt, könnte alternativ auch vorgesehen sein, die den Leukotrien-Inhibitor umfassende pharmazeutische Zusammensetzung entsprechend mit einer erhöhten Konzentration bzw. Menge an Leukotrien-Inhibitor herzurichten bzw. den Leukotrien-Inhibitor in entsprechend erhöhter Dosierung zu verabreichen, um die erhöhte Abbaurate zu kompensieren.

Als Begleittherapie kann hierbei grundsätzlich vorgesehen sein, dass alle Patienten mit bronchopulmonaler Dysplasie zusätzlich gemäß den üblichen Therapieprinzipien behandelt werden. Die Standard-Therapie der BPD beinhaltet je nach Schweregrad der BPD folgende Schritte, die einzeln oder in beliebiger Kombination ergänzend durchgeführt werden können:
- Prävention mit pränataler, systemischer Glukokortikoidgabe bei der Mutter;
- Sauerstoffapplikation;
- Flüssigkeitsrestriktion;
- Diuretika (Hydrochlorothiazid, Spironolacton bzw. Furosemid);
- erhöhte Energiezufuhr;
- Inhalationstherapie (Salbutamol bzw. Ipratropiumbromid);
- systemischen Gabe von Glukokortikoiden;
- Vermeidung eines Barotraumas der Lunge durch möglichst schonende Beatmung;
- frühzeitiges Ausweichen auf CPAP-Beatmung;
- großzügige Indikationsstellung zur Surfactantsubstitution;
- Vitamin A-Gabe;
- Gabe von Bronchodilatatoren; und
- Antibiotikatherapie.

Von allen Patienten, die an dem Heilversuch teilnehmen, wurden in den ersten acht Wochen nach Therapiebeginn routinemäßig Daten erfasst. Im weiteren Verlauf erstreckte sich die Dokumentation - soweit möglich - auf alle vier Wochen. Die angegebenen Erfassungszeiträume lagen bei den zweiwöchentlichen Untersuchungen in einem Zeitfenster von ±3 Tagen, bei den monatlichen Kontrollen lag der Erfassungszeitraum bei ±7 Tagen.

Folgende Parameter wurden bei den Visiten der leichten BPD erfasst:
1. Körperliche Untersuchung und Körpergewicht, Körpergröße, Blutdruck, Herzfrequenz, Allgemeinzustand;
2. Peripherer Sauerstoffgehalt des Blutes (SpO₂); und
3. Verweildauer im Krankenhaus in Tagen.

Folgende Parameter werden bei den Visiten der moderaten und schweren BPD erfasst:
1. Körperliche Untersuchung und Körpergewicht, Blutdruck (Systolischer-/Diastolischer- und Mitteldruck), Herzfrequenz, Allgemeinzustand;
2. Peripherer Sauerstoffgehalt des Blutes (SpO₂);
3. FiO₂ (fakultativ);
4. Arterielle/Kapilläre Blutgasanalyse (pH, pCO2, pO₂, BE) (fakultativ);
5. Beatmungsform (fakultativ);
6. Beatmungsdruck (Spitzendruck, Mitteldruck) (fakultativ);
7. PEEP (fakultativ);
8. Beatmungsdauer in Tagen;
9. Röntgen Thorax (fakultativ);
10. Verweildauer im Krankenhaus in Tagen; und
11. Versterben (Zeitpunkt des Todes, Alter in Lebenstagen).

Es gilt zu beachten, dass oben genannte Parameter ausschließlich aus bereits vorliegenden Untersuchungen erfasst, nie jedoch zusätzlich, d.h. außerhalb von Routinebestimmungen erhoben wurden.

Die Endauswertung erfolgte sowohl bei der moderaten BPD (FiO₂ < 0,30 mit 36 Wochen PMA oder bei Entlassung nach Hause) als auch bei der schweren BPD (FiO₂ > 0,30 und/oder Beatmung bzw. CPAP mit 36 Wochen PMA oder bei Entlassung nach Hause) ein halbes Jahr jeweils nach Abschluss der Therapiephase. Die beiden Behandlungsgruppen sowie die jeweilige Gruppe ohne zusätzliche Therapie mit einem Leukotrien-Inhibitor wie beispielsweise Montelukast wurden bezüglich folgender Parameter (falls vorhanden) verglichen:
1. Dauer des Krankenhausaufenthaltes (d);
2. Beatmungsdauer (d);
3. Beatmungsform;
4. Beatmungsdruck (Spitzen-/ Mitteldruck) (mmHg);
5. PEEP (mmHg);
6. Peripherer Sauerstoffgehalt des Blutes (SpO₂);
7. Arterieller/Kapilläre pCO₂ (mmHg);
8. Röntgen Thorax (Stadien I - IV n. Northway);
9. Körpergewicht (kg), Blutdruck (mmHg), Herzfrequenz (/min);
10. Tod (Zeitpunkt des Todes, Alter in Lebenstagen);
11. PAS-Wert; und
12. Auftreten/Art unerwünschter Ereignisse.

Diese Parameter sollten grundsätzlich mindestens gleich bleiben oder den entsprechenden Parametern bei Anwendung der bisherigen Therapieform überlegen sein. Eine Auswertung erfolgte anhand von klinischen Aspekten sowie anhand der Berechnung und des Vergleichs des PAS-Wertes. Mit Hilfe Nichtparametrischer Testverfahren (Wilcoxon-Test für Matchpaare) wurde die PAS-Werte der Wochen 2, 4, 6, 8, 12, 16, 20 und 24 mit Woche 0 sowie untereinander verglichen und auf Signifikanz überprüft.

Bei vier solchen Patienten im Gewicht zwischen 460 g - 890 g wurde der Heilversuch mit einer pharmazeutischen Zusammensetzung, welche Montelukast als Leukotrien-Inhibitor umfasste, durchgeführt. Dabei wurde Montelukast im Rahmen des Heilversuchs in einer Dosis von 1-2 mg/kg Körpergewicht/d in der vorstehend beschriebenen Weise über 3-12 Wochen verabreicht. Zu diesem Zweck wurde die pharmazeutische Zusammensetzung als Mini-Tabletten hergerichtet, da bei einem Gewicht zwischen 0,45 kg und 0,89 kg eine Tagesdosierung von 2 x 0,25 mg bis zu 2 x 0,45 mg benötigt wurde. Bei keinem der behandelten Patienten traten Nebenwirkungen auf. Bei allen Kindern besserte sich die Lungenfunktion. Zwei Kinder verstarben dennoch später nach Absetzen des Medikaments an der Erkrankung, zwei Patienten wurden vollständig geheilt. In einem Kollektiv mit erwarteter 100% Letalität stellt dies eine jedoch einen erheblichen und überraschenden Behandlungserfolg dar.

## Patentansprüche

1. Verwendung von Montelukast und/oder Zafirlukast und/oder Pranlukast zur Herstellung einer pharmazeutischen Zusammensetzung zur Prophylaxe und/oder Behandlung von bronchopulmonaler Dysplasie bei frühgeborenen Kindern.

2. Verwendung nach Anspruch 1, bei welcher die pharmazeutische Zusammensetzung zur oralen und/oder intravenösen und/oder aerogenen und/oder rektalen Verabreichung hergerichtet wird.

3. Pharmazeutische Zusammensetzung zur Anwendung in der Prophylaxe und/oder Behandlung von bronchopulmonaler Dysplasie bei frühgeborenen Kindern, wobei die pharmazeutische Zusammensetzung Montelukast und/oder Zafirlukast und/oder Pranlukast umfasst.

4. Pharmazeutische Zusammensetzung nach Anspruch 3 zur Anwendung in der Prophylaxe und/oder Behandlung von bronchopulmonaler Dysplasie bei frühgeborenen Kindern, welche zur Verabreichung von Montelukast und/oder Zafirlukast und/oder Pranlukast in einer Dosierung zwischen (0,2± 10%) mg/kg/d und (3,5± 10%) mg/kg/d, insbesondere zwischen (1,0± 10%) mg/kg/d und (2,0± 10%) mg/kg/d, hergerichtet ist.

5. Pharmazeutische Zusammensetzung nach einem der Ansprüche 3 oder 4 zur Anwendung in der Prophylaxe und/oder Behandlung von bronchopulmonaler Dysplasie bei frühgeborenen Kindern, welche eine geeignete Menge wenigstens eines pharmazeutisch akzeptablen Trägerstoffs umfasst.

## Claims

1. Use of montelukast and/or zafirlukast and/or pranlukast in the manufacture of a pharmaceutical composition for the prophylaxis and/or treatment of bronchopulmonary dysplasia in prematurely born infants.

2. The use according to claim 1 wherein the pharmaceutical composition is prepared for oral and/or intravenous and/or aerogenic and/or rectal use.

3. Pharmaceutical composition for use in the prophylaxis and/or treatment of bronchopulmonary dysplasia in prematurely born infants, wherein the pharmaceutical composition comprises motelukast and/or zafirlukast and/or pranlukast.

4. The pharmaceutical composition according to claim 3 for use in the prophylaxis and/or treatment of bronchopulmonary dysplasia in prematurely born infants, wherein the pharmaceutical composition is prepared for the administration of montekulast and/or zafirlukast and/or pranlukast in a dosage between (0.2 ± 10 %) mg/kg/d and (3.5 ± 10 %) mg/kg/d, in particular between (1.0 ± 10 %) mg/kg/d and (2.0 ± 10 %) mg/kg/d.

5. The pharmaceutical composition according to any one of claims 3 and 4 for use in the prophylaxis and/or treatment of bronchopulmonary dysplasia in prematurely born infants, wherein the pharmaceutical composition comprises a suitable amount of at least one pharmaceutically acceptable carrier substance.

## Revendications

1. Utilisation de montélukast et / ou de zafirlukast et / ou de pranlukast, destiné à la fabrication d'une composition pharmaceutique, destinée à la prophylaxie et / ou au traitement de la dysplasie bronchopulmonaire chez les enfants prématurés.

2. Utilisation selon la revendication 1, pour lequel la composition pharmaceutique est préparée en vue de l'administration orale et / ou intraveineuse et / ou aérogène et / ou rectale.

3. Composition pharmaceutique, destinée à une application dans la prophylaxie et / ou le traitement de la dysplasie bronchopulmonaire chez les enfants prématurés, la composition pharmaceutique comprenant le montélukast et / ou le zafirlukast et / ou le pranlukast.

4. Composition pharmaceutique selon la revendication 3, destinée à être appliquée dans la prophylaxie et / ou le traitement de la dysplasie bronchopulmonaire chez les enfants prématurés, laquelle est préparée aux fins d'administration de montélukast et / ou de zafirlukast et / ou de pranlukast, dans un dosage entre (0,2 ± 10 %) mg/kg/d et (3,5 ± 10 %) mg/kg/d, en particulier entre (1,0 ± 10 %) mg/kg/d et (2,0 ± 10 %) mg/kg/d.

5. Composition pharmaceutique selon l'une des revendications 3 ou 4, destinée à être appliquée dans la prophylaxie et / ou le traitement de la dysplasie bronchopulmonaire chez les enfants prématurés, laquelle comprend une quantité adéquate au moins d'un excipient, acceptable sur le plan pharmaceutique.
